# EUROPEAN PATENT SPECIFICATION

(11) **EP 2 069 782 B1**
(45) Date of publication and mention of the grant of the patent: **29.12.2010**
(21) Application number: 07803258.8
(22) Date of filing: 05.09.2007
(51) Int. Cl.: G01N 33/50, A61K 39/00

(54) **METHOD FOR DETERMINING THE QUANTITY AND QUALITY OF HYBRIDOMAS**
VERFAHREN ZUR BESTIMMUNG DER QUANTITÄT UND QUALITÄT VON HYBRIDOMEN
PROCÉDÉ DE DÉTERMINATION DE LA QUANTITÉ ET DE LA QUALITÉ D'HYBRIDOMES

(30) Priority: 05.09.2006 SI 200600207; 23.01.2007 EP 07101015
(43) Date of publication of application: 17.06.2009
(73) Proprietor: Celica, Biomedical Center, d.o.o., 1000 Ljubljana (SI)
(72) Inventor: ZOREC, Robert, 6630 Piran (SI); KREFT, Marko, 1000 Ljubljana (SI); GABRIJEL, Mateja, 8210 Trebnje (SI)
(74) Representative: von Kreisler Selting Werner
(86) International application number: PCT/EP2007/059296
(87) International publication number: WO 2008/028929

(56) References cited:
- WO-A-2005/063961
- US-A1- 2003 082 163
- GABRIJEL M ET AL: "Quantification of lysosomal fusion in electrofused hybridoma cells" BOOK OF ABSTRACTS, [Online] March 2006 (2006-03), XP002441360 Kranjska gora, Slovenia Retrieved from the Internet: URL:http://www.nib.si/file/41876/file.html > [retrieved on 2007-07-06] cited in the application
- HAYASHI T ET AL: "Immunogenicity and therapeutic efficacy of dendritic-tumor hybrid cells generated by electrofusion" CLINICAL IMMUNOLOGY, ACADEMIC PRESS,, US, vol. 104, no. 1, July 2002 (2002-07), pages 14-20, XP002963801 ISSN: 1521-6616
- ORENTAS R J ET AL: "Electrofusion of a weakly immunogenic neuroblastoma with dendritic cells produces a tumor vaccine" CELLULAR IMMUNOLOGY, ACADEMIC PRESS, SAN DIEGO, CA, US, vol. 213, no. 1, 10 October 2001 (2001-10-10), pages 4-13, XP002963600 ISSN: 0008-8749 cited in the application
- GABRIJEL M ET AL: "Quantification of cell hybridoma yields with confocal microscopy and flow cytometry" BIOCHEMICAL AND BIOPHYSICAL RESEARCH COMMUNICATIONS, ACADEMIC PRESS INC. ORLANDO, FL, US, vol. 314, no. 3, 13 February 2004 (2004-02-13), pages 717-723, XP004485025 ISSN: 0006-291X cited in the application
- GONG J ET AL: "INDUCTION OF ANTITUMOR ACTIVITY BY IMMUNIZATION WITH FUSIONS OF DENDRITIC AND CARCINOMA CELLS" NATURE MEDICINE, NATURE PUBLISHING GROUP, NEW YORK, NY, US, vol. 3, no. 5, May 1997 (1997-05), pages 558-561, XP002910553 ISSN: 1078-8956 cited in the application
- LUZIO J P ET AL: "Relationship between endosomes and lysosomes" BIOCHEMICAL SOCIETY TRANSACTIONS, vol. 29, no. 4, August 2001 (2001-08), pages 476-480, XP002455145 ISSN: 0300-5127
- MATSUMOTO SACHIKO ET AL: "Allogeneic gastric cancer cell-dendritic cell hybrids induce tumor antigen (carcinoembryonic antigen) specific CD8(+) T cells." CANCER IMMUNOLOGY, IMMUNOTHERAPY : CII FEB 2006 LNKD- PUBMED:15891883, vol. 55, no. 2, February 2006 (2006-02), pages 131-139, ISSN: 0340-7004
- GODDARD R V ET AL: "In vitro dendritic cell-induced T cell responses to B cell chronic lymphocytic leukaemia enhanced by IL-15 and dendritic cell-B-CLL electrofusion hybrids." CLINICAL AND EXPERIMENTAL IMMUNOLOGY JAN 2003 LNKD- PUBMED:12519390, vol. 131, no. 1, January 2003 (2003-01), pages 82-89, ISSN: 0009-9104
- PARKHURST MARIA R ET AL: "Hybrids of dendritic cells and tumor cells generated by electrofusion simultaneously present immunodominant epitopes from multiple human tumor-associated antigens in the context of MHC class I and class II molecules." JOURNAL OF IMMUNOLOGY (BALTIMORE, MD. : 1950) 15 MAY 2003 LNKD- PUBMED:12734382, vol. 170, no. 10, 15 May 2003 (2003-05-15) , pages 5317-5325, ISSN: 0022-1767
- GONG J ET AL: "Activation of antitumor cytotoxic T lymphocytes by fusions of human dendritic cells and breast carcinoma cells." PROCEEDINGS OF THE NATIONAL ACADEMY OF SCIENCES OF THE UNITED STATES OF AMERICA 14 MAR 2000 LNKD- PUBMED:10688917, vol. 97, no. 6, 14 March 2000 (2000-03-14) , pages 2715-2718, ISSN: 0027-8424
- TREVOR KATRINA T ET AL: "Generation of dendritic cell-tumor cell hybrids by electrofusion for clinical vaccine application." CANCER IMMUNOLOGY, IMMUNOTHERAPY : CII AUG 2004 LNKD- PUBMED:15048588, vol. 53, no. 8, August 2004 (2004-08), pages 705-714, ISSN: 0340-7004
- DUNN W A ET AL: "Low temperature selectively inhibits fusion between pinocytic vesicles and lysosomes during heterophagy of 125I-asialofetuin by the perfused rat liver." THE JOURNAL OF BIOLOGICAL CHEMISTRY 25 JUN 1980 LNKD- PUBMED:6155379, vol. 255, no. 12, 25 June 1980 (1980-06-25), pages 5971-5978, ISSN: 0021-9258
- HAYLETT T ET AL: "Endosome-lysosome fusion at low temperature." THE JOURNAL OF BIOLOGICAL CHEMISTRY 5 MAY 1991 LNKD- PUBMED:2022649, vol. 266, no. 13, 5 May 1991 (1991-05-05), pages 8322-8327, ISSN: 0021-9258

## Description

### Field of the invention

The present invention belongs to the field of cell biology, therapeutic biotechnology and immunology and it consists of a method for detection and quantification of hybridoma cells produced by the fusion of eukaryotic parental cells. The invention focuses on the fusion between dendritic and tumor cells.

The present invention relates to a method employing confocal microscopy for the assessment of the yield of hybridoma cells by means of monitoring the fusion status of differently labelled subcellular organelles, for example late endocytic compartments. In the past lysosomes (a form of late endocytic organelles) were thought to take part in antigen presentation and it was considered that hybridoma production may be monitored by studying specifically lysosomal fusion in hybridoma cells (Gabrijel M., Kreft M., Zorec, R. (2006) Quantification of lysosomal fusion in electrofused hybridoma cells, Book of Abstracts, Kranjska gora, Slovenija, p79). However, it is now generally accepted that delivery of endocytosed macromolecules to lysosomes occurs by content mixing between late endosomes and lysosomes (Paul R. Pryor et al. 2000, The Role of Intraorganellar Ca2+ in Late Endosome-Lysosome Heterotypic Fusion and in the Reformation of Lysosomes from Hybrid Organelles The Journal of Cell Biology, 149: 1053-1062). In fact in mammalian cells, the organelles of the late endocytic pathway have been shown to interact with each other and to be in dynamic equilibrium both in vivo and in vitro. Thus, content mixing and/or exchange of membrane proteins has been observed between late endosomes, lysosomes, and between late endosomes and lysosomes.

The present description discloses the dynamics of late endocytic compartments as the object of antigen presentation in hybridoma cells. Endocytic compartments were fluorescently labelled by fluid phase endocytosis. The invention furthermore provides a method for evaluation of the level of fused late endocytic subcellular compartments in a single hybridoma cell under conditions facilitating heterotypic or homotypic organelle fusion. Late endocytic compartment fusion is a physiological process that takes place in the antigen presentation. The extent of late endocytic compartment fusion therefore indicates the effectiveness of hybridoma cell vaccine against cancer. Hybridomas consist of dendritic and tumor cells: namely represent a cellular vaccine that can be used in general for the modulation of the immune response. The assessment of the amount and the immunogenicity of hybridoma cells is an important step in the production of hybridomas.

The amount of hybridoma cells assessed by the method of the invention comprises detection and quantification of late endocytic compartments. Late endocytic compartments originate from parental cells derived from human or/and animal sources. The physiological process of late endocytic compartments is associated with the complex system of antigen presentation in antigen presenting cells, for example in dendritic cells. Therefore, endocytic compartment fusion events are anticipated in hybridomas consisting of antigen presenting (e.g. dendritic) and tumor cells. Specifically, for the tumor antigens to be presented by the dendritic cells, both forming a hybridoma cell, the MHC class II-peptide complexes are expected to be formed between MHC class II molecules which are primarily located in late endocytic compartments of dendritic cells and tumor peptides from late endocytic compartments of tumor cells. Thus, due to the property of endocytic compartments of fusing with one another, known and unknown tumor antigens are expressed on the surface of hybridoma cells. Higher imunogenicity of hybridomas is of key importance for the effectiveness of cellular vaccine against cancer in the patient's body.

The present invention can be used for labelling and determination of the number of hybridomas for antibody production and for monitoring subcellular organelle dynamics.

It is of key importance to have an optimal approach for rapid and objective detection and quantification of hybridoma cells. In the recent past, researchers have relied primarily on flow cytometry to enumerate dually fluorescent hybridoma cells that contain red and green cytoplasmic fluorescent dyes. But it was reported that flow cytometry is not the only method for the detection and assessment of hybridoma cells, since aggregated cells can also appear as hybridomas and affect the results detected by flow cytometry. Furthermore, the inappropriate colour compensation and staining of cells with lipid-soluble dyes can result in artefact splay in the dually staining quadrant. To eliminate these problems a sensitive, rapid and objective confocal microscopy based method for the detection and quantification of electrofused cells was developed (Gabrijel et al., Quantification of cell hybridoma yields with confocal microscopy and flow cytometry, Biochem. Biophys. Res. Commun., vol. 314, year 2004, pages 717-723). However, in both techniques the labeling of cytoplasm of both parental cell types by red or green fluorescent dyes was typically employed. The results of these methods give the information only about the amount of hybridoma cells in the electrofused sample. In the past, investigators have reported successful cell fusions with relatively high efficiency rates, which were determined after few hours of incubation at 37°C (Orentas et al., Electrofusion of a weakly immunogenic neuroblastoma with dendritic cells produces a tumor vaccine, Cell Immunol., vol. 213, year 2001, pages 4-13).

According to the literature, the transport of tumor antigens to the cell surface is relatively slow and usually requires more than a few hours at 37°C to represent the whole spectrum of tumor associated antigens on their surface (Pierre et al., Developmental regulation of MHC class II transport in mouse dendritic cells, Nature, vol. 388, 1997, notebook 6644, pages 787-792; Roosnek E et al., Kinetics of MHC-antigen complex formation on antigen-presenting cells, J. Immunol., vol. 140, 1988, notebook 12, pages 4079-4082). Short postfusion incubation time can therefore result in a weak immunogenicity of hybridomas and lower effectiveness of the hybridoma cell vaccine. Therefore it is of key importance to develop a new approach for the quantification of hybridoma cells in a fusion sample based on the observation of the dynamics of antigen presentation.

US 2003/0082163 discloses a method and apparatus for electrofusing a plurality of cancer cell types to dendritic cells (DCs) which results in the formation of electrofused DC-tumor cells with high effiency and high viability. The method comprises subjecting the cells with multiple pulses of voltage in specially designed media. Evidence demonstrates those cells contain all essential DC molecules and express tumor antigens. A single vaccination of animals with pre-existing tumors results in substantial eradication of tumors and cure of animals. The electrofused-cells can be used immediately. These fusion hybrids are to be used for the treatment of cancer directly as vaccines or indirectly by activating immune lymphocytes for adoptive immunotherapy.

Hayashi, T. et al. disclose in Clinical Immunology Vol. 104, No. 1, July, pp. 14-20, 2002 that dendritic cells (DCs) are potent antigen-presenting cells capable of inducing strong immune responses to weak tumor-associated antigens. Among various DC-based approaches, cancer immunotherapy with DC-tumor fusion hybrids offers advantages of polyclonal stimulation of a diverse array of tumor antigens. However, prevalent fusion methods using chemical fusogens such as polyethylene glycol often result in toxicity and low fusion efficiency. In this article, the authors describe an electrofusion technique, applicable to processing large numbers of cells with consistent and high fusion efficiency. Generation of fusion hybrids was verified by unequivocal experimental evidence. In animal models, fusion hybrids expressed the nature DC-like phenotype. They stimulated both CD4 and CD8 tumor-specific T cells to secrete interferon- in vitro. In immunotherapy, a single vaccination with DC-tumor fusion cells along with interleukin-12 as an adjuvant eradictated tumors established in the skin and lung. These results provide an impetus for treating cancer patients with similary generated cells.

### Background of the invention

Dendritic cell based vaccines are considered as a promising strategy to stimulate host immunity to recognize and eliminate malignant cells. The most promising design of dendritic cell based vaccines are hybridomas, that are produced by the fusion of dendritic and tumor cells. The potential advantage that distinguishes this approach from other dendritic cell strategies is that hybridomas present known and unknown tumor antigens on their surface in the context with costimulatory molecules and MHC molecules class II and I that arise from dendritic cells. Antigen presentation results in the induction of a correspondingly balanced cytotoxic and T -helper cell responses (Gong et al., Induction of antitumor activity by immunization with fusions of dendritic and carcinoma cells, Nat. Med., vol. 3, year 1997, pages 558 - 561). The activation of T-helper cells is crucial for effective and durable antitumor immunity. Clinical trials have demonstrated that hybridomas are able to stimulate tumor specific immunity, however only a subset of patients experienced disease regression or stabilization. There are many reasons underlying the low efficiency of hybridoma tumor vaccines.

The immunogenicity of hybridoma tumor vaccine depends on the amount of hybridoma cells and on their quality: i.e. the tumor antigens represented on the surface of hybridomas. However, to understand the latter process it critically requires an insight into the role of late endocytic compartments, such as lysosomes in hybridoma cell production.

Today researchers rely primarily on flow cytometry to enumerate dually fluorescent hybridoma cells that contain red and green cytoplasmic fluorescent dyes (Jaroszeski et al., Flow cytometric detection and quantitation of cell-cell electrofusion products, Methods Mol. Biol., vol. 91, 1998, pages 149-156). The number of hybridoma cells is determined usually after the electrofusion process by detection of dually fluorescent cells that contain the cytoplasm with the mixture of red and green cytoplasmic fluorescent dyes. But it was reported that flow cytometry is the method that is not sensitive enough for the objective detection and assessment of hybridoma cells, since aggregated cells can also appear as hybridomas and affect the results detected by flow cytometry (Hayashi et al., Immunogenicity and therapeutic efficacy of dendritic-tumor hybrid cells generated by electrofusion, Clin. Immunol., vol.104, pages 14-20). Furthermore, the results of this method give the information only about the amount of hybridoma cells in the electrofused sample.

In the past, investigators reported successful cell fusions with relatively high efficiency rates, which were determined after a few hours of incubation at 37°C (Orentas et al., 2001 Electrofusion of a weakly immunogenic neuroblastoma with dendritic cells produces a tumor vaccine, Cell Immunol., vol. 213, year 2001, pages 4-13). However, as described previously the transport of tumor antigens to the cell surface is relatively slow and usually requires more than few hours at 37°C to represent the whole spectrum of tumor associated antigens on their surface. Short postfusion incubation time can therefore result in weak immunogenicity of hybridomas and lower effectiveness of the hybridoma cell vaccine (Orentas et al., 2001 Electrofusion of a weakly immunogenic neuroblastoma with dendritic cells produces a tumor vaccine, Cell Immunol., vol. 213, year 2001, pages 4-13). Therefore it is another object of the invention to provide a new approach for the quantification of hybridoma cells in a fusion sample based on the observation of the dynamics of antigen presentation. Considering that late endocytic compartments are involved in antigene presentation (Gabrijel M., Kreft M., Zorec, R. (2006) Quantification of lysosomal fusion in electrofused cells, Book of Abstracts, Kranjska gora, Slovenija, p79), it is imperative to know the conditions that facilitate late endocytic compartment fusion in hybridomas.

### Summary of the invention

A technical problem of the previously described method by flow cytometry is solved by the invention developed for labeling, monitoring and quantitative image analysis of late endocytic compartments by laser confocal microscopy Late endocytic compartments, which also include lysosomes, are dynamic organelles that fuse with each other and are involved in antigen presentation in dendritic cells and in hybridomas as well. If in a hybridoma cell, generated by fusion of two donor, dendritic and tumor cells is supposed to present antigens of both parental cell types on the common cell hybridoma surface, then fusion of late endocytic compartments is anticipated. Indeed, in the present invention a method for quantification of hybridoma cells in a fusion sample was introduced, which is based on the monitoring of the dynamics of late endocytic compartments, which take part in the antigen presentation pathway. Hybridomas are determined by detection of fluorescently labelled (usually red and green) late endocytic compartments, which are directly involved in the antigen presentation process. The fusion of late endocytic compartments that originate from different parental cell types is observed under specific incubation parameters, such as temperature and time of incubation.

Late endocytic compartment fusion in hybridomas is a physiological process, which enables complex formation between molecules of the major histocompatibility complex from late endocytic compartments of dendritic cells and tumor peptides from late endocytic compartments of tumor cells.

For this reason the aim of the present invention is the development of a method for the optimization of the preparation of hybridoma cell vaccines based on the intracellular physiological process of antigen presentation.

The invention provides a method for detection of the amount and quality of hybridoma cells consisting of eukaryotic cells by confocal microscopy, the method comprising the steps of
(a) staining of late endocytic compartments of origin for cell-lines derived from human or animal sources, in particular wherein the late endocytic compartments are lysosomes,
(b) both parental cell types are separately stained by different fluorescent dyes,
(c) after staining, cells are fused for a at least 2h incubation time at a temperature of from 25 °C to 35 °C.
(d) the extent of late endocytic compartment fusion is determined by confocal microscopy.

The method of the invention includes:
- labelling subcellular organelles, specifically late endocytic compartments that are involved in antigen presentation pathway;
- quantification of the number of late endocytic compartments in a single cell by confocal microscopy;
- determination of the yield of hybridoma cells by detection of fluorescently labelled (usually red and green) late endocytic compartments that originate from both parental cell types;
- determination of the extent of fused late endocytic compartments in a hybridoma cell under specific incubation conditions by quantitative confocal microscopy and image analysis.

The data of the analysis provide the information about the dynamics of the antigen presentation pathway, which is of key importance for the immunogenicity of hybridomas and the effectiveness of a cellular vaccine against cancer in the patient' s body as well.

According to one embodiment of the invention the incubation time is of from 2 to 30 hours.

In a further embodiment, the method of the invention is comprising
- a) labelling of late endocytic compartments and the determination of the minimal number of late endocytic compartments per single parental cell by confocal microscopy;
- b) fusion of both parental cell types and incubation at 25 to 35°C for 2 to 30 hours;
- c) analysis of fusion samples for the presence of hybridoma cells by confocal microscopy;
- d) determination of the number of hybridoma cells that contain at least the minimum number of late endocytic compartments that originate from separate parental cell types and that are stained separately by different fluorescent dyes;
- e) determination of the amount of hybridoma cells expressed as the ratio between the number of hybridoma cells and the number of all fluorescent cells detected by confocal microscopy.

According to one embodiment of the invention the following steps are sequentially performed:
- labelling subcellular organelles, specifically late endocytic compartments that are involved in antigen presentation pathway;
- quantification of the number of late endocytic compartments in a single cell by confocal microscopy;
- determination of the yield of hybridoma cells by detection of fluorescently labelled, for example red and green, late endocytic compartments that originate from both parental cell types;
- determination of the extent of fused late endocytic compartments in a hybridoma cell by quantitative confocal microscopy and image analysis.

In a further embodiment of the invention the following steps are sequentially performed:
a) determination of the minimal number of labelled late endocytic compartments per single parental cell;
b) acquiring confocal images of fused and control samples where fusion process was omitted;
c) acquiring series of confocal images to optically cut the cell and obtain three dimensional images, for example *z*-stacks, for each detected hybridoma cell;
d) determination of the number of unfused late endocytic compartments that originate from each parental cell type, respectively, and determination of the number of fused late endocytic compartments;
e) determination of the number of hybridoma cells in each fusion sample based on the minimal number of late endocytic compartments, labelled with two different colours, for example red and green, that originate from each parental cell type and minimal number of fused late endocytic compartments, for example yellow.

According to one embodiment of the invention in step a) laser confocal *z*-stacks of images are acquired for each randomly selected fluorescent molecules loaded, non-electrofused cell.

According to one embodiment of the invention, the number of labeled late endocytic compartments per cell was counted with the proviso that each late endocytic compartment can appear in a predetermined number of adjacent optical slices, an assessment of the number of late endocytic compartments is performed for each cell population, respectively and the minimum number of late endocytic compartments per cell is assessed.

The invention pertains further to a method wherein in step b) after fusion process samples are incubated at 25 to 35 °C for 2 to 30 hours and thereafter are analyzed by confocal images for the presence of hybridoma cells, the confocal images are acquired by laser confocal microscopy, in particular wherein green fluorescence is excited by argon and red fluorescence by helium/neon laser.

In a further embodiment in step c) fusion samples are analyzed for the presence of hybridoma cells and optical slices of *z*-stacks are acquired by confocal microscopy for each detected hybridoma cell.

According to one embodiment of the invention in step d) the number of green, red and yellow late endocytic compartments is evaluated by computer assistance.

In one embodiment of the method of the invention the hybridomas are defined as cells that contain at least the minimum number of red, green or yellow fluorescent late endocytic compartments, or pairs of other fluorescent markers and the number of fused late endocytic compartments is indicative for the level of the immunogenicity of the hybridoma.

In one embodiment, step e) of the method of the invention involves counting of hybridomas in a fusion sample and the expression of the level of hybridoma cells in a fusion sample as the ratio between the number of hybridoma cells and the number of fluorescently labelled cells in the fusion sample.

According to one embodiment of the method of the invention the hybridoma cells are formed by the fusion between tumor cells and eukaryotic cells, for example, dendritic cells.

The incubation temperature can also be about 27 °C. For practical reasons the incubation time may be over night.

In contrast to hybridoma cell quantification methods of prior art where cytoplasm of cells was typically stained, or the method of labelling specifically lysosomes, the method of the invention provides labelling and evaluation of the late endocytic compartments that are involved in tumor antigen presentation, which are the measure of the quality of hybridoma cells. Fused endocytic compartments are competent of antigen presentation, since antigens from different endocytic compartments may get processed for antigen presentation. Relatively high yield in endocytic compartment fusion is proportional to the quality of hybridomas.The number of late endocytic compartments can only be detected by confocal microscopy. Therefore, one embodiment of the invention is a novel confocal microscopy method that in contrast to flow cytometry (where adjacent but not fused cells may appear as hybridomas) gives not only the information about the amount of hybridoma cells but can also be used for the determination of the quality of hybridoma cells in a cell suspension.

The invention provides a method for quantification of hybridoma cells based on the detection of subcellular structures (late endocytic compartments) that are directly involved in antigen presentation pathway of hybridoma cell. The invention utilizes a characteristic of homotypic or heterotypic subcellular organelle fusion. Fusion of subcellular organelle from different cell types can also be detected.

Late endocytic compartments are e. g. stained by fluorescent dextran molecules that enter the cell through endocytic pathway. Each parental cell type, for instance dendritic and tumor cells, is labeled by a different fluorescent dye, respectively, usually red and green fluorescent dyes can be used. Transport of fluorescent molecules lasts several to at least 20 hours and more at 37°C, depending on the cell type. Cells are incubated in fluorescent molecule-free culture media for several hours at 37°C in order to remove fluorescent molecules from early endosomal compartments.

This invention provides a fundamental method for the quantification of hybridoma cells in a cellular vaccine and for the determination of the activity of antigen presenting pathway in hybridoma cells under different incubation conditions.

Another embodiment of the invention includes several sequential steps:
a) determination of the minimal number of labelled late endocytic compartments per single parental cell;
b) acquiring confocal images of fused and control samples where fusion process was omitted;
c) acquiring series of confocal images to optically cut the cell and obtain three dimensional images (*z*-stacks) of each detected hybridoma cell;
d) determination of the number of unfused late endocytic compartments that originate from each parental cell type, respectively, and determination of the number of fused late endocytic compartments;
e) determination of the number of hybridoma cells in each fusion sample based on the minimal number of red and green late endocytic compartments that originate from each parental cell type and minimal number of fused yellow (red and green) late endocytic compartments;
in which in
step a) laser confocal *z*-stacks of images are acquired for each randomly selected fluorescent molecule loaded, non-electrofused cell. The number of labeled late endocytic compartments per cell was counted considering the possibility that each late endocytic compartment can appear in three adjacent optical slices. The assessment of the number of late endocytic compartments is performed for each cell population, respectively. Finally, the minimum number of late endocytic compartments per cell is assessed;
step b) after fusion process samples are incubated at 25 to 35°C or for 2 to 30 hours and then they are analyzed for the presence of hybridoma cells by confocal images that are acquired by laser confocal microscopy. Green fluorescence is excited by argon and red fluorescence by helium / neon laser;
step c) fusion samples are analyzed for the presence of hybridoma cells and optical slices of *z*-stacks are acquired by confocal microscopy for each detected hybridoma cell;
step d) the number of green, red and yellow (fused) late endocytic compartments is evaluated using the computer software. Hybridomas are defined as cells that contain at least the minimum number of red, green or yellow fluorescent lysosomes. The number of yellow (fused) late endocytic compartments may be an indicator for the level of the immunogenicity of hybridoma;
step e) involves counting of hybridomas in a fusion sample and the expression of the level of hybridoma cells in a fusion sample as the ratio between the number of hybridoma cells and the number of fluorescently labelled cells in fusion sample.

For the preparation of hybridoma cell vaccine autologic tumor cells can be used as one parental cell population. These cells are fused and prepared as described above prior to application into the patient.

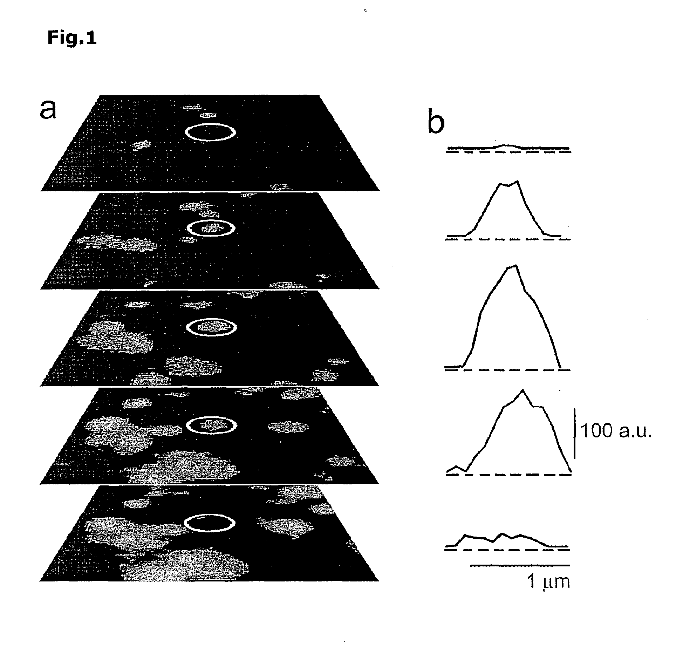

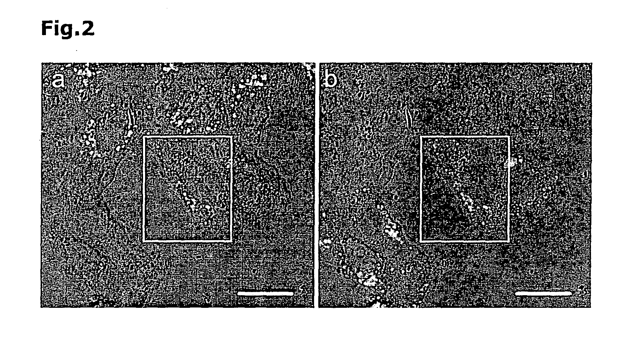

The description discloses a method that is described in more detail by Figure 1 and Figure 2.
Figure 1: Determination of the number of late endocytic compartments in a cell. To estimate the number of late endocytic compartments per cell *z*-stacks of dextran loaded CHO cells were acquired. Labeled late endocytic compartments were counted considering the possibility that a late endocytic compartment can appear in more than one optical slice. (a) A series of vertical optical slices. The thickness of the optical slices was 0.5 µm, The circles indicate a selected late endocytic compartment through which a line intensity profile was drawn in (b). The series of graphs represent line intensity profiles indicating that an image of a late endocytic compartment may span over three optical slices.
Figure 2: A confocal image represents an electrofused sample of a mixture of cells. The frame highlights a hybridoma cell, determined as a cell that contains at least 30 red and 30 green or 30 yellow (red and green) fluorescent late endocytic compartments and two nuclei, marked by N. (a) A confocal image shows the overlay of a DIC and red channels. White regions that are localized in some cells represent late endocytic compartments that are labelled with red fluorescent dextran molecules. (b) The overly of DIC and green channels. Late endocytic compartments loaded with green fluorescent dextrans are shown as small clusters of white pixels. Note that fused late endocytic compartments in the hybridoma cell appear as white area in both confocal images (a) and (b). Scale bar: 20 µm.

The description is further describing in more detail the following, which is however not part of the invention:

Both parental cell types (dendritic and tumor cells) are incubated 4 to 20 hours at 37 °C with red and green fluorescent dyes. The dye uptake is stopped by rinsing the cell cultures with the culture medium. In order to clear the marker from endocytic compartments, cells are incubated for additional several hours at 37°C in marker- free medium. Next we acquire confocal *z-*stacks of images in several randomly selected fluorescently loaded, non-electrofused cells (Figure 1 a). The average number of labeled late endocytic compartments per single cell is counted by computer software, considering the possibility that each late endocytic compartments can appear in more than one optical slice of the acquired *z*-stack (Figure 1 b). The same number of red and green labeled cells is then electrofused and afterwards incubated for 20 hours at 37°C. Fusion samples are then analyzed for the presence of hybridoma cells by images acquired by laser confocal microscopy (Figure 2 a, 2 b). Cells are identified as hybridomas when they contain at least a minimum number of red, green, or yellow (red and green) fluorescent late endocytic compartments. The number of late endocytic compartments in a cell was evaluated by generating *z*-stacks of randomly selected non-electrofused dextran labeled cells, subjected to 3D counting by computer software, assuming that each late endocytic compartment may appear in three optical slices.

The amount of yellow (fused) late endocytic compartments in a single hybridoma cells is expressed by the number of yellow pixels relative to all fluorescent pixels on each optical slice of the acquired *z*-stack. The average percentage of electrofused hybridoma cells in each fusion sample was expressed by the ratio between the number of hybridoma cells and the number of fluorescent cells detected by confocal microscopy.

## Claims

1. A method for detection of the amount and quality of hybridoma cells consisting of eukaryotic cells by confocal microscopy, the method comprising the steps of
(a) staining of late endocytic compartments of origin for cell-lines derived from human or animal sources, in particular wherein the late endocytic compartments are lysosomes,
(b) both parental cell types are separately stained by different fluorescent dyes.
(c) after staining, cells are fused for a at least 2h incubation time at a temperature of from 25 °C to 35 °C.
(d) the extent of late endocytic compartment fusion is determined by confocal microscopy.

2. The method of claim 1, wherein the incubation time is of from 2 to 30 hours.

3. The method of any one of the claims 1 or 2 comprising
- a) labelling of late endocytic compartments and the determination of the minimal number of late endocytic compartments per single parental cell by confocal microscopy;
- b) fusion of both parental cell types and incubation at 25 to 35°C for 2 to 30 hours;
- c) analysis of fusion samples for the presence of hybridoma cells by confocal microscopy;
- d) determination of the number of hybridoma cells that contain at least the minimum number of late endocytic compartments that originate from separate parental cell types and that are stained separately by different fluorescent dyes;
- e) determination of the amount of hybridoma cells expressed as the ratio between the number of hybridoma cells and the number of all fluorescent cells detected by confocal microscopy.

4. The method any one of the claims 1 to 3, wherein the following steps are sequentially performed:
- labelling subcellular organelles, specifically late endocytic compartments that are involved in antigen presentation pathway;
- quantification of the number of late endocytic compartments in a single cell by confocal microscopy;
- determination of the yield of hybridoma cells by detection of fluorescently labelled, for example red and green, late endocytic compartments that originate from both parental cell types;
- determination of the extent of fused late endocytic compartments in a hybridoma cell by quantitative confocal microscopy and image analysis.

5. The method of any one of the claims 1 to 4 wherein the following steps are sequentially performed:
a) determination of the minimal number of labelled late endocytic compartments per single parental cell;
b) acquiring confocal images of fused and control samples where fusion process was omitted;
c) acquiring series of confocal images to optically cut the cell and obtain three dimensional images, for example *z*-stacks, for each detected hybridoma cell;
d) determination of the number of unfused late endocytic compartments that originate from each parental cell type, respectively, and determination of the number of fused late endocytic compartments;
e) determination of the number of hybridoma cells in each fusion sample based on the minimal number of late endocytic compartments, labelled with two different colours, for example red and green, that originate from each parental cell type and minimal number of fused late endocytic compartments for example yellow.

6. The method of claim 5 wherein in step a) laser confocal *z*-stacks of images are acquired for each randomly selected fluorescent molecules loaded, non-electrofused cell.

7. The method of claim 6 wherein the number of labeled late endocytic compartments per cell was counted with the proviso that each late endocytic compartment can appear in a predetermined number of adjacent optical slices, an assessment of the number of late endocytic compartments is performed for each cell population, respectively and the minimum number of late endocytic compartments per cell is assessed.

8. The method of claim 5 wherein in step b) after fusion process samples are incubated at 25 to 35 °C for 2 to 30 hours and thereafter are analyzed by confocal images for the presence of hybridoma cells, the confocal images are acquired by laser confocal microscopy, in particular wherein green fluorescence is excited by argon and red fluorescence by helium/neon laser.

9. The method of claim 5 wherein in step c) fusion samples are analyzed for the presence of hybridoma cells and optical slices of *z*-stacks are acquired by confocal microscopy for each detected hybridoma cell.

10. The method of claim 5 wherein in step d) the number of green, red and yellow late endocytic compartments is evaluated by computer assistance.

11. The method of claim 10 wherein the hybridomas are defined as cells that contain at least the minimum number of red, green or yellow fluorescent late endocytic compartments, or pairs of other fluorescent markers, and the number of fused late endocytic compartments is indicative for the level of the immunogenicity of the hybridoma.

12. The method of claim 5 wherein step e) involves counting of hybridomas in a fusion sample and the expression of the level of hybridoma cells in a fusion sample as the ratio between the number of hybridoma cells and the number of fluorescently labelled cells in the fusion sample.

13. The method of any one of the claims 1 to 12 wherein the hybridoma cells are formed by the fusion between tumor cells and eukaryotic cells, for example, dendritic cells.

## Patentansprüche

1. Verfahren zum Nachweis der Menge und Qualität von aus eukaryontischen Zellen bestehenden Hybridomzellen durch konfokale Mikroskopie, wobei das Verfahren die folgenden Schritte umfasst:
(a) Anfärben später endozytotischer Ursprungskompartimente zur Darstellung von Zelllinien, die aus humanen oder tierischen Quellen stammen, wobei die späten endozytotischen Kompartimente insbesondere Lysosomen sind;
(b) beide parentalen Zelltypen werden getrennt durch verschiedene Fluoreszenzfarbstoffe angefärbt;
(c) nach dem Anfärben werden die Zellen während einer Inkubationszeit von wenigstens 2 h bei einer Temperatur von 25 °C bis 35 °C fusioniert;
(d) das Ausmaß der Fusion der späten endozytotischen Kompartimente wird durch konfokale Mikroskopie bestimmt.

2. Verfahren gemäß Anspruch 1, wobei die Inkubationszeit 2 bis 30 Stunden beträgt.

3. Verfahren gemäß einem der Ansprüche 1 oder 2, umfassend:
- a) Markieren später endozytotischer Kompartimente und Bestimmung der minimalen Anzahl später endozytotischer Kompartimente pro einzelner parentaler Zelle durch konfokale Mikroskopie;
- b) Fusion beider parentaler Zelltypen und Inkubation bei 25 bis 35 °C während 2 bis 30 Stunden;
- c) Analyse von Fusionsproben auf die Anwesenheit von Hybridomzellen durch konfokale Mikroskopie;
- d) Bestimmung der Anzahl der Hybridomzellen, die wenigstens die minimale Anzahl später endozytotischer Kompartimente enthalten, welche von getrennten parentalen Zelltypen stammen und die getrennt durch verschiedene Fluoreszenzfarbstoffe angefärbt sind;
- e) Bestimmung der Menge an Hybridomzellen, ausgedrückt als Verhältnis der Anzahl der Hybridomzellen zur Anzahl aller fluoreszierenden Zellen, die durch konfokale Mikroskopie nachgewiesen wurden.

4. Verfahren gemäß einem der Ansprüche 1 bis 3, wobei die folgenden Schritte nacheinander durchgeführt werden:
- Markieren subzellulärer Organellen, insbesondere später endozytotischer Kompartimente, die am Antigenpräsentationsweg beteiligt sind;
- Quantifizierung der Anzahl später endozytotischer Kompartimente in einer einzelnen Zelle durch konfokale Mikroskopie;
- Bestimmung der Ausbeute an Hybridomzellen durch Nachweis fluoreszenzmarkierter, zum Beispiel roter und grüner, später endozytotischer Kompartimente, die von beiden parentalen Zelltypen stammen;
- Bestimmung des Ausmaßes der fusionierten späten endozytotischen Kompartimente in einer Hybridomzelle durch quantitative konfokale Mikroskopie und Bildanalyse.

5. Verfahren gemäß einem der Ansprüche 1 bis 4, wobei die folgenden Schritte nacheinander durchgeführt werden:
a) Bestimmung der minimalen Anzahl markierter später endozytotischer Kompartimente pro einzelner parentaler Zelle;
b) Erfassung konfokaler Bilder von fusionierten Proben und Kontrollproben, bei denen der Fusionsvorgang weggelassen wurde;
c) Erfassung von Reihen konfokaler Bilder als optische Schnitte der Zelle und Gewinnung dreidimensionaler Bilder, zum Beispiel Z-Stapel, für jede nachgewiesene Hybridomzelle;
d) Bestimmung der Anzahl unfusionierter später endozytotischer Kompartimente, die von jedem parentalen Zelltyp stammen, und Bestimmung der Anzahl fusionierter später endozytotischer Kompartimente;
e) Bestimmung der Anzahl der Hybridomzellen in jeder Fusionsprobe auf der Basis der minimalen Anzahl später endozytotischer Kompartimente, die mit zwei verschiedenen Farben, zum Beispiel Rot und Grün, markiert sind und die von jedem parentalen Zelltyp stammen, und der minimalen Anzahl fusionierter später endozytotischer Kompartimente, zum Beispiel Gelb.

6. Verfahren gemäß Anspruch 5, wobei in Schritt a) laserkonfokale Z-Stapel von Bildern für jede zufällig ausgewählte, mit fluoreszierenden Molekülen beladene nichtelektrofusionierte Zelle erfasst werden.

7. Verfahren gemäß Anspruch 6, wobei die Anzahl der markierten späten endozytotischen Kompartimente pro Zelle bestimmt wurde, mit der Maßgabe, dass jedes späte endozytotische Kompartiment in einer vorbestimmten Anzahl von benachbarten optischen Schnitten erscheinen kann, eine Bewertung der Anzahl später endozytotischer Kompartimente für jede Zellpopulation durchgeführt wird und die minimale Anzahl später endozytotischer Kompartimente pro Zelle bewertet wird.

8. Verfahren gemäß Anspruch 5, wobei in Schritt b) nach dem Fusionsvorgang Proben 2 bis 30 Stunden lang bei 25 bis 35 °C inkubiert und danach anhand von konfokalen Bildern bezüglich der Anwesenheit von Hybridomzellen analysiert werden, die konfokalen Bilder durch konfokale Lasermikroskopie erfasst werden, wobei insbesondere grüne Fluoreszenz durch Argonlaser und rote Fluoreszenz durch Helium/Neon-Laser angeregt wird.

9. Verfahren gemäß Anspruch 5, wobei in Schritt c) Fusionsproben auf die Anwesenheit von Hybridomzellen analysiert werden und für jede nachgewiesene Hybridomzelle durch konfokale Mikroskopie optische Schnitte von Z-Stapeln erfasst werden.

10. Verfahren gemäß Anspruch 5, wobei in Schritt d) die Anzahl grüner, roter und gelber später endozytotischer Kompartimente computerunterstützt bewertet wird.

11. Verfahren gemäß Anspruch 10, wobei die Hybridome definiert sind als Zellen, die wenigstens die minimale Anzahl an rot, grün oder gelb fluoreszierenden späten endozytotischen Kompartimenten oder Paare anderer Fluoreszenzmarker enthalten, und die Anzahl fusionierter später endozytotischer Kompartimente das Ausmaß der Immunogenität des Hybridoms anzeigt.

12. Verfahren gemäß Anspruch 5, wobei Schritt e) das Zählen von Hybridomen in einer Fusionsprobe und das Ausdrücken der Menge an Hybridomzellen in einer Fusionsprobe als Verhältnis der Anzahl an Hybridomzellen zur Anzahl fluoreszenzmarkierter Zellen in der Fusionsprobe beinhaltet.

13. Verfahren gemäß einem der Ansprüche 1 bis 12, wobei die Hybridomzellen durch die Fusion zwischen Tumorzellen und eukaryontischen Zellen, zum Beispiel dendritischen Zellen, gebildet werden.

## Revendications

1. Procédé de détection de la quantité et de la qualité de cellules d'hybridome consistant en des cellules eucaryotes par microscopie confocale, le procédé comprenant les étapes de
(a) coloration de compartiments endocytaires tardifs d'origine pour des lignées cellulaires dérivées de sources humaines ou animales, où notamment les compartiments endocytaires tardifs sont des lysosomes,
(b) coloration séparée des deux types de cellule parentale par des teintes fluorescentes différentes,
(c) après coloration, fusion des cellules pendant un temps d'incubation d'au moins 2 h à une température de 25 °C à 35°C,
(d) détermination de l'ampleur de la fusion des compartiments endocytaires tardifs par microscopie confocale.

2. Procédé selon la revendication 1, dans lequel le temps d'incubation est de 2 à 30 heures.

3. Procédé selon l'une quelconque des revendications 1 ou 2, comprenant
- a) le marquage de compartiments endocytaires tardifs et la détermination du nombre minimal de compartiments endocytaires tardifs par cellule parentale individuelle par microscopie confocale ;
- b) fusion des deux types de cellule parentale et incubation à 25 à 35°C pendant 2 à 30 heures ;
- c) analyse des échantillons de fusion quant à la présence de cellules d'hybridome par microscopie confocale ;
- d) détermination du nombre de cellules d'hybridome qui contiennent au moins le nombre minimal de compartiments endocytaires tardifs ayant pour origine des types de cellule parentale séparés et qui sont colorées séparément par des teintes fluorescentes différentes ;
- e) détermination de la quantité de cellules d'hybridome exprimées comme le rapport entre le nombre de cellules d'hybridome et le nombre de toutes les cellules fluorescentes détectées par microscopie confocale.

4. Procédé selon l'une quelconque des revendications 1 à 3, dans lequel les étapes suivantes sont effectuées séquentiellement :
- marquage d'organelles sous-cellulaires, spécifiquement de compartiments endocytaires tardifs qui sont impliqués dans la voie de présentation de l'antigène ;
- quantification du nombre de compartiments endocytaires tardifs dans une cellule individuelle par microscopie confocale ;
- détermination de la production de cellules d'hybridome par détection de compartiments endocytaires tardifs marqués de façon fluorescente, par exemple en rouge et vert, qui ont pour origine les deux types de cellule parentale ;
- détermination de l'ampleur de compartiments endocytaires tardifs fusionnés dans une cellule d'hybridome par microscopie confocale quantitative et analyse d'image.

5. Procédé selon l'une quelconque des revendications 1 à 4, dans lequel les étapes suivantes sont effectuées séquentiellement :
a) détermination du nombre minimal de compartiments endocytaires tardifs marqués par cellule parentale individuelle ;
b) acquisition d'images confocales d'échantillons fusionnés et témoins où un processus de fusion a été omis;
c) acquisition de séries d'images confocales pour couper optiquement la cellule et obtenir des images en trois dimensions, par exemple des piles Z, pour chaque cellule d'hybridome détectée ;
d) détermination du nombre de compartiments endocytaires tardifs non fusionnés qui ont pour origine chaque type de cellule parentale, respectivement, et détermination du nombre de compartiments endocytaires tardifs fusionnés ;
e) détermination du nombre de cellules d'hybridome dans chaque échantillon de fusion en se basant sur le nombre minimal de compartiments endocytaires tardifs, marqués avec deux couleurs différentes, par exemple rouge et vert, qui ont pour origine chaque type de cellule parentale et le nombre minimal de compartiments endocytaires tardifs fusionnés, par exemple jaune.

6. Procédé selon la revendication 5, dans lequel dans l'étape a), des piles Z confocales laser d'images sont acquises pour chaque cellule non électrofusionnée chargée de molécules fluorescentes choisies au hasard.

7. Procédé selon la revendication 6, dans lequel le nombre de compartiments endocytaires tardifs marqués par cellule a été compté à condition que chaque compartiment endocytaire tardif puisse apparaître dans un nombre prédéterminé de tranches optiques adjacentes, une estimation du nombre de compartiments endocytaires tardifs est effectuée pour chaque population de cellules, respectivement et le nombre minimal de compartiments endocytaires tardifs par cellule est estimé.

8. Procédé selon la revendication 5, dans lequel à l'étape b), après le processus de fusion, des échantillons sont incubés à 25 à 35 °C pendant 2 à 30 heures, puis sont analysés par des images confocales quant à la présence de cellules d'hybridome, les images confocales sont acquises par microscopie confocale laser, où notamment la fluorescence verte est excitée par l'argon et la fluorescence rouge est excitée par un laser à hélium/néon.

9. Procédé selon la revendication 5, dans lequel à l'étape c), des échantillons de fusion sont analysés quant à la présence de cellules d'hybridome et des tranches optiques de piles Z sont acquises par microscopie confocale pour chaque cellule d'hybridome détectée.

10. Procédé selon la revendication 5, dans lequel à l'étape d), le nombre de compartiments endocytaires tardifs verts, rouges et jaunes est évalué par assistance par ordinateur.

11. Procédé selon la revendication 10, dans lequel les hybridomes sont définis comme des cellules qui contiennent au moins le nombre minimal de compartiments endocytaires tardifs fluorescents rouges, verts ou jaunes, ou des paires d'autres marqueurs fluorescents, et le nombre de compartiments endocytaires tardifs fusionnés est indicatif du niveau d'immunogénicité de l'hybridome.

12. Procédé selon la revendication 5, dans lequel l'étape e) implique le comptage d'hybridomes dans un échantillon de fusion et l'expression du niveau de cellules d'hybridome dans un échantillon de fusion comme le rapport entre le nombre de cellules d'hybridome et le nombre de cellules marquées de façon fluorescente dans l'échantillon de fusion.

13. Procédé selon l'une quelconque des revendications 1 à 12, dans lequel les cellules d'hybridome sont formées par la fusion entre des cellules de tumeur et des cellules eucaryotes, par exemple des cellules dendritiques.
